# FASCICULE DE BREVET EUROPEEN

(11) **EP 2 201 354 B1**
(45) Date de publication et mention de la délivrance du brevet: **07.11.2018**
(21) Numéro de dépôt: 08857647.5
(22) Date de dépôt: 24.09.2008
(51) Int. Cl.: G01N 27/12

(54) **TRANSDUCTEUR A SEMI CONDUCTEURS, ET SON UTILISATION DANS UN CAPTEUR D'ESPECES DONNEUSES OU ACCEPTRICES D'ELECTRONS.**
AUF EINEM HALBLEITER BASIERENDER MESSWANDLER, UND DESSEN VERWENDUNG IN EINEM SENSOR FÜR REDUZIERENDE ODER OXIDIERENDE GASKOMPONENTEN
SEMI-CONDUCTOR TRANSDUCER, AND THE USE THEREOF IN AN ELECTRON DONOR- OR ELECTRON ACCEPTOR-TYPE SENSOR

(30) Priorité: 15.10.2007 FR 0707209
(43) Date de publication de la demande: 30.06.2010
(73) Titulaire: Université Pierre et Marie Curie (Paris 6), 75005 Paris (FR); Centre National de la Recherche Scientifique, 75016 Paris (FR)
(72) Inventeur: BOUVET, Marcel, F-77000 La Rochette (FR); PARRA, Vicente, F-47010 Valladolid (ES)
(74) Mandataire: Goulard, Sophie
(86) Numéro de dépôt international: PCT/FR2008/001325
(87) Numéro de publication internationale: WO 2009/071774

(56) Documents cités:
- EP-A- 0 286 307
- EP-A- 1 085 319
- EP-A- 1 235 070
- EP-A- 1 790 977
- WO-A-01/91202
- WO-A-96/00383
- US-A- 4 871 680
- BOUVET M ET AL: "Molecular Semiconductor-Based Gas Sensors" ENCYCLOPEDIA OF SENSORS, X, XX, vol. 6, 1 janvier 2006 (2006-01-01), pages 227-269, XP009094472 cité dans la demande
- RODRIGUEZ-MENDEZ M L ET AL: "Lutetium bisphthalocyanine thin films as sensors for volatile organic components (VOCs) of aromas" SENSORS AND ACTUATORS B, ELSEVIER SEQUOIA S.A., LAUSANNE, CH, vol. 58, no. 1-3, 21 septembre 1999 (1999-09-21), pages 544-551, XP004253060 ISSN: 0925-4005
- BRUNET ET AL: "Improvement in real time detection and selectivity of phthalocyanine gas sensors dedicated to oxidizing pollutants evaluation" THIN SOLID FILMS, ELSEVIER-SEQUOIA S.A. LAUSANNE, CH, vol. 490, no. 1, 21 octobre 2005 (2005-10-21), pages 28-35, XP005034821 ISSN: 0040-6090
- HO K-C ET AL: "NO2 gas sensing based on vacuum-deposited TTF-TCNQ thin films" SENSORS AND ACTUATORS B, ELSEVIER SEQUOIA S.A., LAUSANNE, CH, vol. 93, no. 1-3, 1 août 2003 (2003-08-01), pages 370-378, XP004437125 ISSN: 0925-4005
- DAS A ET AL.: "A Nitrogen Dioxide Sensor Based on an Organic Transistor Constructed from Amorphous Semiconducting Polymers" ADVANCED MATERIALS, vol. 19, no. 22, 19 novembre 2007 (2007-11-19), pages 4018-4023, XP002542050

## Description

La présente invention concerne un transducteur à semi conducteurs, et son utilisation dans un capteur d'espèces donneuses d'électrons ou acceptrices d'électrons.

On connaît divers dispositifs pour la détection de molécules gazeuses, notamment ceux qui comprennent un transducteur à semi-conducteurs tel que par une couche d'un polymère organique tel que le polypyrrole comme décrit par exemple dans EP 0 286 307, du type résistor, transistor ou diode.

Il existe de nombreux types de diodes ayant divers types de matériaux semi conducteurs dont quelques exemples ont été utilisés comme capteurs chimiques. On connaît notamment par R. Dobulans, J. Latvels, I. Muzikante, E. Fonavs, A. Tokmakov, M. Bouvet ["Molecular diode for gas sensing", Proceedings of the NENAMAT Mobilization Workshop "Nanomaterials and Nanotechnologies", 30-31 mars, Riga, 2005, 130] une diode dont les deux couches semiconductrices sont constituées par deux matériaux moléculaires différents, respectivement de type p et de type n. Cependant, la géométrie et le fonctionnement de ce dispositif ne sont pas optimisés pour son utilisation en tant que capteur chimique, et sa mise en forme est complexe.

M. Bouvet et A. Pauly (Encyclopedia of Sensors, Ed. C.A. Grimes, E.C. Dickey and M.V. Pishko, vol. 6, p. 227-269) décrivent des transducteurs du type résistor et du type transistor à effet de champ, dans lesquels l'élément sensible est un matériau moléculaire électroactif. De tels matériaux sont décrits notamment par J. Simon, J.-J. André, A. Skoulios ["Molecular materials. I : Generalities", Nouv. J. Chim., 1986, 10, 295-311] et par J. Simon, P. Bassoul ["Design of Molecular Materials", Wiley, Chichester, 2000].

Un transistor à effet de champ est constitué par la succession de couches suivantes : un substrat conducteur (par exemple Si dopé), une couche isolante (par exemple SiO₂ ou Si₃N₄), une couche de monophtalocyanine métallée (MPc). La couche MPc est connectée à une électrode source et une électrode drain, et le substrat conducteur est connecté à une électrode grille. La couche MPc constitue à la fois la couche sensible et le matériau dans lequel circule le courant mesuré qui peut être modulé par la tension appliquée sur l'électrode grille. Le matériau moléculaire peut être un polymère semi-conducteur, une phtalocyanine (substituée ou non), une porphyrine (substituée ou non) un oligothiophène (substitué ou non), un pentacène, un fullérène, ou un dérivé de pérylène. Ce type de dispositif est décrit par exemple dans la demande de brevet EP 1 235 070. Un tel dispositif a de bonnes performances pour la détection de molécules. Cependant, son élaboration implique des étapes technologiques compliquées.

Un détecteur de gaz du type résistor comprend, par exemple, un substrat en alumine sur l'une des faces duquel sont placées deux électrodes en Pt interdigitées par-dessus lesquelles est déposé un film de phtalocyanine de cuivre (PcCu) comme décrit par exemple par Brunet et al. (Thin Solid Films, 2005, 490, 28-35). L'autre face du substrat comprend des résistances de Pt pour le chauffage du substrat. La conductivité de PcCu augmente avec la teneur en O₃ ou en NO₂ d'un mélange gazeux avec lequel PcCu est en contact. L'inconvénient d'un tel dispositif réside d'une part dans l'absence de sélectivité par rapport aux molécules gazeuses à détecter, et d'autre part dans la limitation des performances.

La demande de brevet EP 1 790 977 décrit un capteur chimique comprenant un subtrat supportant une couche sensible à base de nanoparticules ou de nanofibres elle-même recouverte d'une couche polymère.

La demande internationale WO 96/00383 décrit une méthode pour déposer des polymères organiques semi-conducteurs sous forme de multicouches sur un support ainsi que l'application de cette méthode à la fabrication de capteurs d'espèce gazeuses.

Les inventeurs ont trouvé que, de manière suprenante, le dépôt, sur la surface sensible d'un resistor à semi conducteur, d'un film d'un matériau semi conducteur différent choisi selon des critères bien déterminés, rendait le transducteur sélectif vis-à-vis des molécules à détecter et améliorait de façon substantielle les performances en tant que capteur, par création d'une hétéroj onction particulière.

Le but de la présente invention est de fournir un capteur d'espèces donneuses d'électrons ou acceptrices d'électrons qui a des performances nouvelles et améliorées par rapport aux capteurs de l'art antérieur. Ce capteur fonctionne à température ambiante contrairement à nombre de capteurs antérieurs.

La présente invention a pour objet un transducteur à semi conducteur, ainsi qu'un capteur d'espèces donneuses d'électrons ou acceptrices d'électrons qui contient un tel transducteur.

Un transducteur selon la présente invention est défini dans la revendication 1. Il est constitué par un substrat isolant à la surface duquel sont déposées deux électrodes et un élément sensible semi-conducteur, et il est caractérisé en ce que :
- l'élément sensible est constitué par une couche d'un matériau moléculaire semi conducteur M1 qui a une conductivité C₁ et une couche d'un matériau moléculaire semi conducteur M2 qui a une conductivité C₂ et une largeur de bande interdite E_{g2} < 1 eV ;
- la couche de matériau M1 est en contact avec les électrodes ;
- la couche de matériau M2 est déposée sur la couche de matériau M1 et n'est pas en contact avec les électrodes ;
- les conductivités sont telles que C₂/C₁ ≥ 1 ;
- le matériau M1 est une monophtalocyanine choisie parmi PcCu, F₈PcCu et F₁₆PcCu; l'α,ω-dihexyl-sexithiophène ou l'acide N,N-dipentyl-pérylène tetracarboxylique diimide.

L'épaisseur de la couche de matériau M1 et l'épaisseur de la couche de matériau M2 sont avantageusement comprises entre 2 et 1000 nm, de préférence entre 20 nm et 100 nm. L'épaisseur des électrodes avantageusement comprise entre 2 nm et 1000 nm.

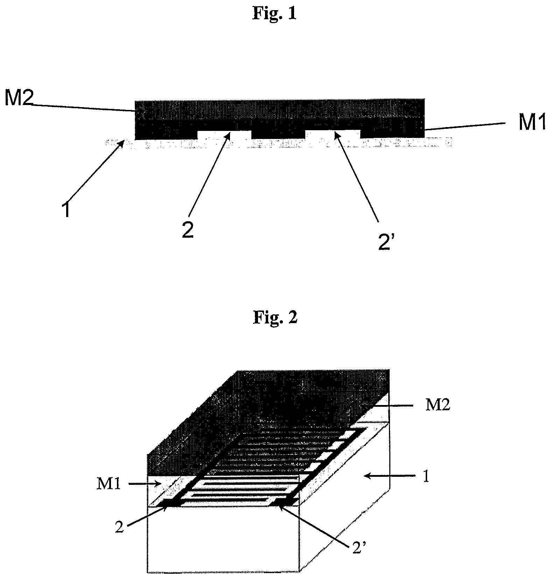

La figure 1 représente une vue schématique d'un transducteur selon l'invention. Le substrat est désigné par la référence 1, les électrodes par les références 2 et 2', et les couches de matériau M1 et M2 respectivement par les références M1 et M2.

Le matériau M2 qui a une largeur de bande interdite E_{g2}<1 eV est Lu(Pc)₂.

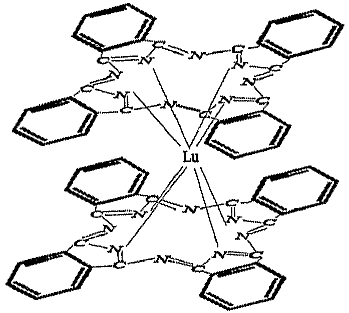

Les bisphtalocyanines sont préparées par un procédé consistant à faire réagir l'ortho-dicyanobenzène avec un sel de lanthanide, soit en solution dans un alcool en présence d'une base organique forte, soit sans solvant, selon les modes opératoires décrits notammment par Kirin I. S., et al., [Russ. J. Inorg. Chem. 1965, 10, 1065-1066] ; par Kirin I. S., et al., [Russ. J. Inorg. Chem. 1967, 12, 369-372] ; par Clarisse C., et al., [Inorg. Chim. Acta 1987, 130, 139-144] et par De Cian A., et al., [Inorg. Chem. 1985, 24, 3162-3167].

Parmi les monophtalocyanines fluorées utilisables comme matériau M1, on peut citer celles qui portent 4 (ne faisant pas partie de l'invention), 8 ou 16 atomes F et qui répondent aux formules suivantes

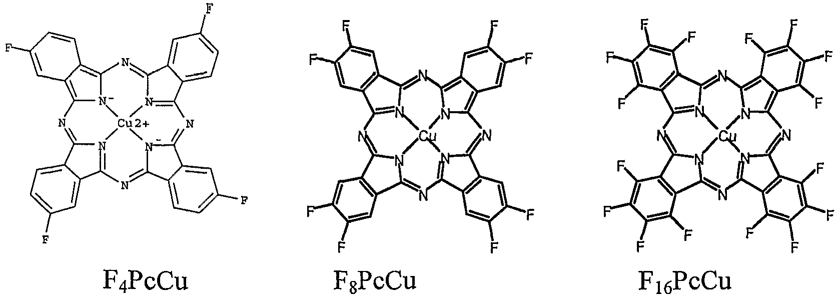

Le degré de fluoration d'une monophtalocyanine a une incidence faible sur Eg, mais entraîne une stabilisation des niveaux électroniques vides et des niveaux électroniques pleins (plus hautes orbitales moléculaires occupées et plus basses orbitales moléculaires inoccupées) avec un décalage quasicontinu de F₀PcCu à F₁₆PcCu de 3,16 à 4,46 eV pour les affinités électroniques et de 5,20 à 6,39 eV pour les potentiels d'ionisation (Cf. R. Murdey, N. Sato, M. Bouvet, Mol. Cryst. Liq. Cryst., 2006, 455, 211-218) par rapport au niveau du vide. Il en résulte qu'un composé M^{II}Pc dans lequel la monophtalocyanine est non fluorée a des propriétés de semi conducteur p, alors qu'un composé M^{II}Pc dans lequel la monophtalocyanine est perfluorée F₁₆Pc a des propriétés de semi conducteur n. Le composé M^{II}F₈Pc a des propriétés intermédiaires et son comportement dépend notamment des électrodes qui font partie du transducteur.

La monophtalocyanine de cuivre CuPc est un produit du commerce, fourni notamment par la société Sigma Aldrich.

Une monophtalocyanine de Cu portant 16 atomes F peut être préparée par réaction du tétrafluoro-1,2-dicyanobenzène avec le métal ou le sel métallique selon le mode opératoire décrit par J. M. Birchall, et al. [J. Chem. Soc., 1970, 2667] ou par D. D. Eley, et al. [J. Chem. Soc., Faraday Trans., 1973, 69, 1808]. La monophtalocyanine perfluorée de Cu est un produit du commerce, fourni par la société Sigma Aldrich.

Une monophtalocyanine de Cu, portant 8 atomes F peut être préparée par réaction du 4,5-difluoro-1,2-dibromobenzène avec un excès de cyanure de cuivre CuCN dans un solvant organique, par exemple DMF, puis en traitant le mélange réactionnel par une solution aqueuse d'ammoniaque concentrée.

Autant que les monophtalocyanines, l'acide N,N-dipentyl-pérylène tetracarboxylique diimide ou le α,ω-dihexyl-sexithiophène sont utilisables à titre de matériau M1.

Le substrat isolant qui porte les électrodes, la couche de matériau M1 ct la couche de matériau M2 peut être constitué par un matériau isolant intrinsèque tel que l'alumine ou le verre, ou par un matériau recouvert d'un matériau diélectrique (par exemple du silicium recouvert par un film de silice ou de nitrure de silicium).

Les électrodes sont constituées par exemple par un film d'or ou par un film d'oxyde d'étain et d'indium (ITO). Elles sont de préférence sous forme d'électrodes interdigitées, tel que représenté notamment sur la figure 2. Les électrodes sont déposées sur le substrat isolant par les procédés classiques, par exemple par évaporation thermique sous vide ou par les techniques de lithographie.

Un transducteur selon l'invention peut être élaboré par un procédé consistant à déposer sur un substrat isolant, les électrodes, la couche de matériau M1, puis la couche de matériau M2.

Une électrode Au peut être déposée par une évaporation thermique.

Une électrode ITO peut être déposée par pulvérisation sous vide.

Le film de matériau M1 peut être déposé par sublimation sous vide, par dépôt à la tournette (généralement désigné par "spin coating") ou par évaporation spontanée de solutions (généralement désignée par "solvent cast").

Le film de matériau M2 peut être déposé par sublimation sous vide, par spin coating ou par solvent cast.

Un capteur d'espèces donneuses ou acceptrices d'électrons qui comprend un transducteur à semi conducteurs selon l'invention est un autre objet de la présente invention; il est défini dans la revendication 8.

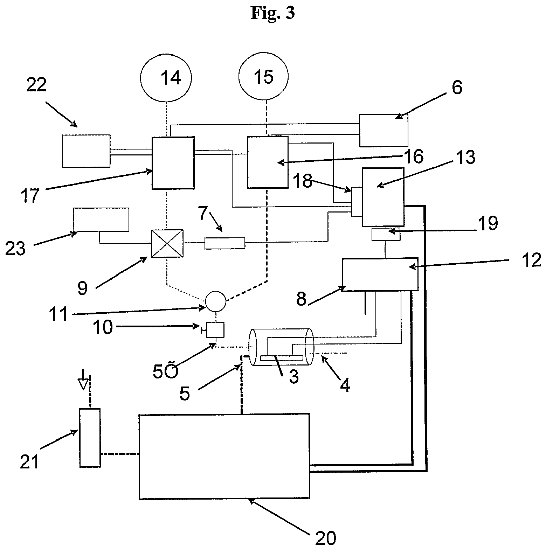

Un mode de réalisation est représenté sur la figure 3, sur laquelle on distingue l'élément sensible dans son enceinte, la partie électronique qui assure la polarisation des dispositifs et le système d'acquisition des données, et le circuit des gaz. Les différents constituants du dispositif sont comme suit :

| | |
|---|---|
| 3 | cellule de détection contenant le transducteur à semi conducteur(s) ; |
| 4 | sortie du gaz de la cellule de détection |
| 5, 5' | entrée du mélange NH₃/Ar, ou du mélange air/ozone dans la cellule de détection |
| 6, 7 | deux diviseurs de tension |
| 8 | thermocouple |
| 9 | électrovanne contrôlée par un temporisateur |
| 10 | soupape de sécurité |
| 11 | mélangeur de flux gazeux |
| 12 | électromètre |
| 13 | ordinateur |
| 14 | source de NH₃ |
| 15 | source de Ar |
| 16, 17 | deux régulateurs de flux gazeux |
| 18 | dispositif analogique d'acquisition de données |
| 19 | dispositif numérique d'acquision de données |
| 20 | analyseur/générateur d'ozone (O341M Environment SA) |
| 21 | filtre de charbon actif |
| 22 | alimentation en courant continu à un potentiel de 0-15 V_{DC} |
| 23 | alimentation en courant continu à un potentiel de -12/+12 V_{DC} |

Les lignes ont les significations suivantes :

| | |
|---|---|
| - | connexions électriques |
| ----- | transfert de gaz/purge (Ar, N₂) |
| ......... | transfert de NH₃ |
| 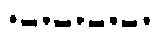 | transfert du gaz à analyser (mélange NH₃/Ar ou air+ozone) vers la cellule de détection 3. |

Un capteur comprenant un transducteur selon la présente invention est particulièrement utile pour la détection d'espèces gazeuses, notamment pour la détection de molécules acceptrices d'électrons telles que O₃ ou NO₂, ou de molécules donneuses d'électrons telles que NH₃.

Lorsqu'un capteur selon l'invention est destiné à la détection de molécules acceptrices d'électrons (ou/et oxydantes), la couche M1 du transducteur est de préférence constituée par CuPc ou par ledit oligothiophène; la monophtalocyanine CuPc et l'α,ω-dihexyl-sexithiophène permettent de bonnes performances.

Lorsqu'un capteur selon l'invention est destiné à la détection de molécules donneuses d'électrons (ou/et réductrices), la couche M1 du transducteur est de préférence constituée par CuF₁₆Pc ou par ledit pérylène diimide; la monophtalocyanine CuF₁₆Pc et l'acide N,N-dipentyl-pérylène tetracarboxylique diimide permettent de bonnes performances.

L'utilisation d'une monophtalocyanine partiellement fluorée dépend de la structure de l'ensemble du transducteur. Lorsque les électrodes sont des électrodes Au ou ITO, un transducteur selon l'invention dans lequel M1 est un composé M^{II}Pc dans lequel la monophtalocyanine est partiellement fluorée (CuF₈Pc) est efficace pour la détection de molécules acceptrices d'électrons.

Le remplacement d'un transducteur à semi conducteurs de l'art antérieur, du type diode, transistor à effet de champ ou résistor, par un transducteur selon la présente invention, fournit un capteur d'espèces donneuses ou acceptrices d'électrons, dont les performances sont substantiellement améliorées, par la présence d'une hétérojonction particulière. La structure même du transducteur le rend adaptable à divers analytes, et permet en particulier une détection sélective et stable dans le temps, à température ambiante. La sélectivité et la stabilité du capteur aux cas particuliers sont obtenues par un choix approprié du matériau formant la couche M1.

La présente invention est décrite plus en détails à l'aide des exemples suivants, auxquels elle n'est cependant pas limitée.

Dans ces exemples, le montage tel que représenté sur la figure 3 décrite ci-dessus a été utilisé pour la détection des espèces gazeuses.

A titre de matériau M1, on a utilisé des phtalocyanines, l'α,ω-dihexyl-sexithiophène (α,ω-dihexyl-6T) ou l'acide N,N-dipentyl-pérylène tetracarboxylique diimide (PTCDI).

Les phtalocyanines utilisées sont PcCu, F₈PcCu, F₁₆PcCu et Lu(Pc)₂.

La monophtalocyanine F₈PcCu a été préparée par réaction du 4,5-difluoro-1,2-dibromobenzène (27,2 g, 0,1 mole) avec le cyanure de cuivre CuCN (26,9 g, 0,3 mole) à 150°C pendant 2 heures dans 50 mL de DMF. Le mélange réactionnel a été traité deux fois par une solution concentrée d'ammoniaque, puis filtré. Le produit solide ainsi récupéré a été lavée par CHCl₃ dans un Soxhlet, puis séché à 100°C sous vide. On a obtenu un fine poudre bleue, avec un rendement de 80%. [composition pour C₃₂H₈F₈N₈Cu :
- calculée: C, 53,38%; H, 1,12%; F, 21,11%; Cu, 8,83%.
- trouvée : C, 52,74%; H, 1,13%; F, 21,03%; Cu, 9,17%

La bisphtalocyanine Lu(Pc)₂ a été préparée par réaction du triacétate de lutécium avec l'ortho dicyanobenzène sans solvant à 300°C.

PcCu et F₁₆PcCu, α,ω-dihexyl-6T et PTCDI sont des produits commercialisés par la société Sigma Aldrich.

### Exemple 1

Cet exemple vise à montrer la différence de comportement de transducteurs du type résistor pour F₈PcCu et Lu(Pc)₂, et du type hétérojonction Lu(Pc)₂ sur F₈PcCu.

### Préparation de résistors

Sur un substrat d'alumine, on a déposé des électrodes de platine par sérigraphie, puis un film de monophtalocyanine. Dans le cas de FgPcCu, un film ayant une épaisseur de 300 nm a été obtenu par sublimation sous vide. Dans le cas de LuPc₂, un film ayant une épaisseur de 100 nm a été obtenu par évaporation spontanée d'une solution.

### Préparation d'une hétérojonction

Sur un substrat d'alumine, on a déposé des électrodes de platine par sérigraphie. Ensuite, on a déposé par évaporation sous vide, successivement un film de F₈PcCu ayant une épaisseur de 300 nm, puis un film Lu(Pc)₂, ayant une épaisseur de 100 nm.

Chacun de ces transducteurs a été testé dans un dispositif tel que représenté sur la figure 3, en faisant passer alternativement un flux d'air sans ozone et un flux d'air contenant environ 700 ppb d'ozone.

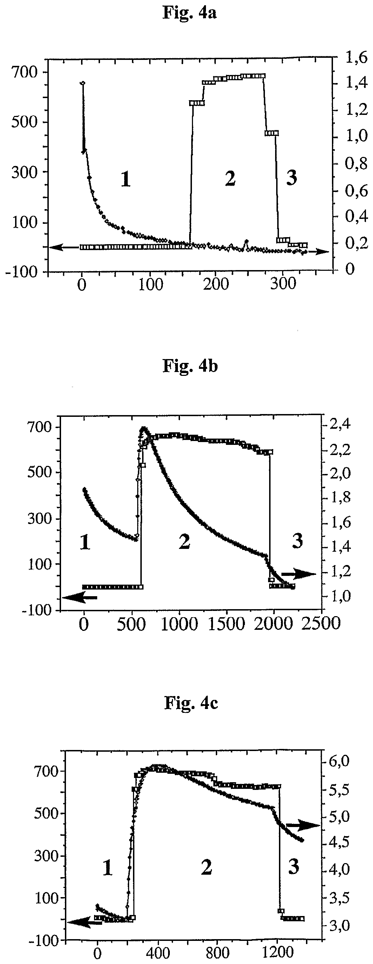

Les figures 4a, 4b et 4c montrent l'évolution du courant I (en A.10¹², sur l'axe des ordonnées, à droite) en fonction du temps (en secondes, sur l'axe des abscisses), c'est-à-dire en fonction de la teneur instantanée en ozone (en ppb, sur l'axe des ordonnées à gauche) dans le flux gazeux. Les zones 1 et 3 correspondent à une atmosphère d'air sans ozone, et la zone 2 correspond à une atmosphère contenant de l'ozone. Chaque flèche indique l'échelle à laquelle se réfère la courbe correspondante.

Sur chacune des figures, la courbe en gris clair montre l'évolution du courant et la courbe en noir montre l'évolution de la composition gazeuse. Il apparaît que :
- le résistor F₈PcCu présente une conductivité très faible, et il ne réagit pas à l'ozone,
- le résistor à Lu(Pc)₂ réagit de manière très nette à l'ozone, mais la réaction n'est pas stable dans le temps ;
- l'hétéroj onction Lu(Pc)₂ sur F₈PcCu présente une conductivité beaucoup plus élevée que la seule couche de F₈PcCu, il réagit de manière significative et la stabilité est considérablement améliorée par rapport à celle d'un résistor Lu(Pc)₂.

Ces résultats montrent clairement l'effet suprenant résultant de l'utilisation d'une hétérojonction dans un transducteur selon l'invention, par rapport aux transducteurs du type resistor de l'art antérieur.

### Exemple 2

Cet exemple vise à montrer la différence de comportement de transducteurs du type hétérojonction Lu(Pc)₂ sur FₓPcCu, x étant 0, 8 ou 16, suivant que le transducteur est soumis à un gaz contenant O₃ ou à un gaz contenant NH₃.

### Transducteurs utilisés pour la détection de O₃

Sur un substrat de silicium recouvert de silice, on a déposé des électrodes de Au par sublimation sous vide, lesdites électrodes étant séparées par 10 µm. Ensuite, on a déposé un film de FₓPcCu par sublimation sous vide, puis un film Lu(Pc)₂, également par sublimation sous vide, les films de phtalocyanine ayant chacune une épaisseur (contrôlée par microbalance à quartz piézoélectrique) de 100 nm. Pour les deux couches phtalocyanine, le dépôt a été effectué à l'aide d'un système VEECO 770, sous une pression de 10⁻⁶ Torr, avec une vitesse de dépôt de 2 Å/seconde.

Chacun des transducteurs ainsi obtenus a été testé dans un montage analogue à celui représenté sur la figure 3 équipé du générateur/analyseur d'ozone, en faisant passer un flux d'air contenant 90 ppb d'ozone, puis un flux d'air propre (filtré sur charbon actif) sans ozone, avec un débit de 1,6 L/min.

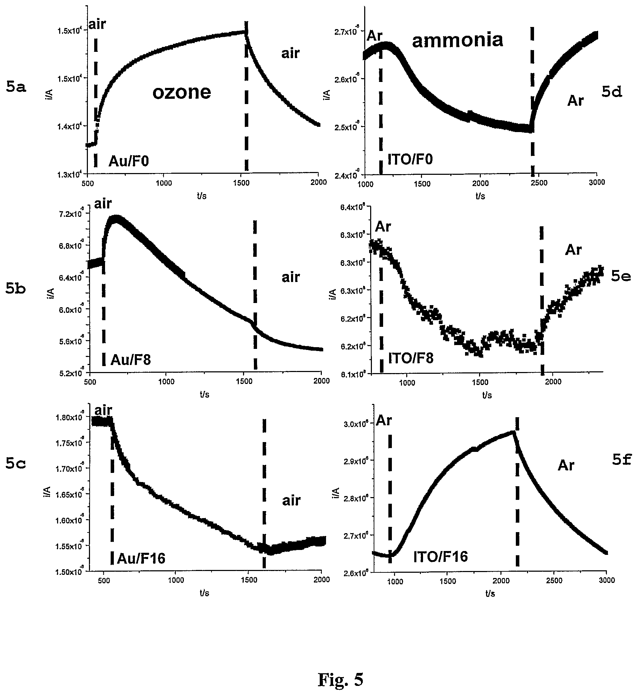

Les figures 5a, 5b et 5c montrent l'évolution du courant I (en ampère) en fonction du temps (en secondes), pour chacun des transducteurs contenant respectivement PcCu, F₈PcCu et F₁₆PcCu.

Ces figures montrent l'intérêt du transducteur utilisé, en particulier influence considérable du matériau M1 sur la réponse du dispositif. En particulier, le courant augmente en présence d'ozone lorsque M1 est PcCu, et diminue lorsque M1 est F₁₆PcCu. PcCu donne la réponse la plus stable vis à vis de l'ozone, avec une bonne sensibilité.

### Transducteurs utilisés pour la détection de NH₃

Sur un substrat de verre, on a déposé par pulvérisation un film de ITO formant les électrodes. Lesdites électrodes sont séparées par 75 µm. Ensuite, on a déposé, dans les mêmes conditions que pour le transducteur pour la détection de O₃, à l'aide d'un système VEECO 770, sous une pression de 10⁻⁶Tor, avec une vitesse de dépôt de 2 Å/seconde, un film de FₓPcCu, puis un film de Lu(Pc)₂, chacun des films de phtalocyanine ayant une épaisseur de 100 nm.

Chacun de ces transducteurs a été testé dans un dispositif analogue à celui de la figure 3, équipé d'un système de régulation par débimètres massiques de flux de NH₃ à partir de bouteilles de NH₃ et de Ar, en faisant passer un flux gazeux contenant 35 ppm de NH₃, puis un flux gazeux sans NH₃, avec un débit de 0,5 L/min.

Les figures 5d, 5e et 5f montrent l'évolution du courant I (en ampère) en fonction du temps (en secondes), respectivement pour le transducteur contenant PcCu, F₈PcCu et F₁₆PcCu.

Ces figures montrent, comme précédemment, l'intérêt du transducteur utilisé, en particulier l'influence considérable du matériau M1 sur la réponse du dispositif. En particulier, le courant augmente en présence de NH₃ lorsque M1 est F₁₆PcCu, et diminue lorsque M1 est PcCu. Ces figures montrent également que l'utilisation de F₁₆PcCu comme matériau M1 est favorable à la détection de NH₃.

### Exemple 3

Cet exemple vise à illustrer un transducteur du type hétérojonction Lu(Pc)₂ sur α,ω-dihexyl-6T, ainsi que la réversibilité de la réponse obtenue lors de son utilisation pour la détection de l'ozone.

Un tel transducteur a été préparé selon le procédé décrit ci-dessus à l'exemple 2 ci-dessus en suivant la procédure décrite pour la préparation des transducteurs utilisés pour la détection de l'ozone ; chacun des films d'α,ω-dihexyl-6T et de Lu(Pc)₂ présentait une épaisseur de 100 nm.

Ce transducteur a ensuite été testé dans un montage analoque à celui représenté sur la figure 3 équipé du générateur/analyseur d'ozone, en faisant passer un flux d'air contenant 400 ppb d'ozone, puis un flux d'air propre (filtre sur charbon actif) sans ozone, avec un débit de 1,6 L/min, selon le protocole suivant :
- trois cycles : flux d'ozone pendant 5 min (exposition) / flux d'air pendant 15 min (repos), puis
- trois cycles : flux d'ozone pendant 2 min / flux d'air pendant 8 min.

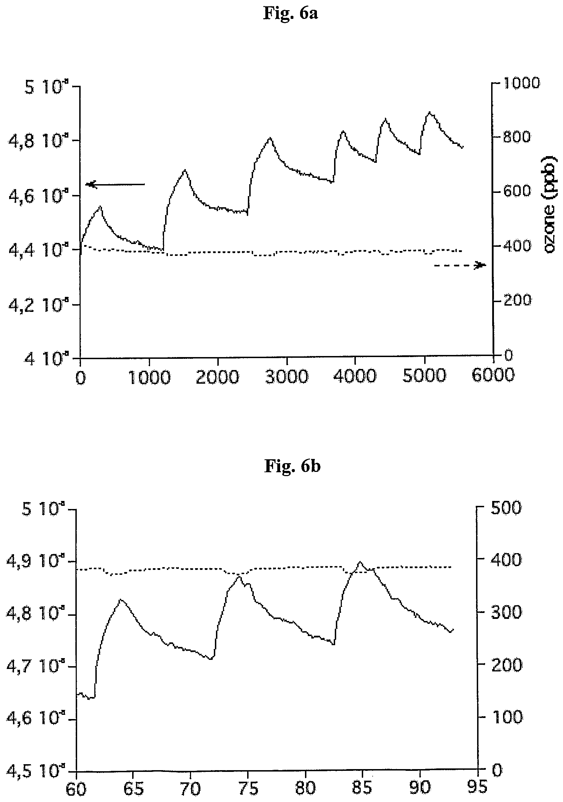

Les résultats obtenus sont donnés sur les figures 6a et 6b annexées qui représentent :
- figure 6a: l'évolution du courant I (en nA sur l'axe des ordonnées à gauche) en fonction du temps (en sec sur l'axe des abscisses), c'est-à-dire en fonction de la teneur instantanée en ozone (en ppb, sur l'axe des ordonnées à droite), dans le flux gazeux. Chaque flèche indique l'échelle à laquelle se réfère la courbe correspondante (courbe en trait plein : intensité du courant ; courbe en trait pointillé : teneur instantannée en ozone).
- la figure 6b : l'évolution du courant I (en nA sur l'axe des ordonnées à gauche) en fonction du temps (en min sur l'axe des abscisses), c'est-à-dire en fonction de la teneur instantannée en ozone (en ppb, sur l'axe des ordonnées à droite), dans le flux gazeux.

La courbe 6a montre que quel que soit le mode opératoire utilisé, la réponse est reproductible ; une meilleure réversibilité du capteur étant néanmoins obtenue avec les cycles les plus courts (exposition 2 min/repos 8 min). La courbe de la figure 6b qui ne montre que les résultats obtenus pendant les cycles courts.

### Exemple 4

Cet exemple vise à illustrer un transducteur du type hétérojonction Lu(Pc)₂ sur PTCDI, ainsi que la réversibilité de la réponse obtenue lors de son utilisation pour la détection de l'ammoniac.

Un tel transducteur a été préparé selon le procédé décrit ci-dessus à l'exemple 2 en suivant la procédure décrite pour la préparation des transducteurs utilisés pour la détection de l'ammoniac ; chacun des films de PTDCI et de Lu(Pc)₂ présentait une épaisseur de 100 nm.

Ce transducteur a ensuite été testé dans un montage analogue à celui représenté sur la figure 3 équipé d'un système de régulation par débimètres massiques de flux de NH₃ à partir de bouteilles de NH₃ et de Ar, en faisant passer un flux de gazeux NH₃/Ar contenant 1000 ppm de NH₃, puis un flux gazeux sans NH₃ avec un débit de 0,5 L/min, selon le protocole suivant :
- trois cycles : flux de NH₃/Ar pendant 5 min (exposition) / flux sans NH₃ pendant 15 min (repos), puis
- trois cycles : flux de NH₃/Ar pendant 2 min / flux sans NH₃ pendant 8 min.

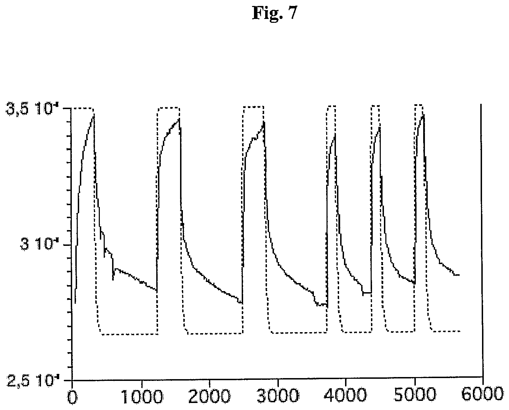

Les résultats obtenus sont donnés sur la figure 7 annexée qui représente l'évolution du courant I (en A sur l'axe des ordonnées) en fonction du temps (en sec sur l'axe des abscisses) et qui correspond à la courbe en trait continu. La courbe en trait pointillé correspond aux cycles d'exposition/repos à NH₃/Ar.

On constate une très bonne réversibilité du capteur quelle que soit la durée des cycles exposition/repos à NH₃/Ar.

## Revendications

1. Transducteur constitué par un substrat isolant (1) à la surface duquel sont déposées deux électrodes (2, 2') et un élément sensible semi conducteur constitué par une couche d'un matériau moléculaire semi conducteur M1 en contact avec les électrodes (2, 2') et une couche d'un matériau moléculaire semi conducteur M2 qui est déposée sur la couche de matériau M1 et n'est pas en contact avec les électrodes (2, 2'), ledit transducteur étant **caractérisé en ce que** :
- le matériau M1 est une monophtalocyanine choisie parmi PcCu, F₈PcCu et F₁₆PcCu ; l'α,ω-dihexyl-sexithiophène ou l'acide N,N-dipentyl-pérylène tetracarboxylique diimide ;
- le matériau M2 est Lu(Pc)₂.

2. Transducteur selon la revendication 1, **caractérisé en ce que** l'épaisseur de chacune des couches de M1, M2 et des électrodes (2, 2') est comprise entre 2 et 1000 nm.

3. Transducteur selon la revendication 1, **caractérisé en ce que** le substrat isolant (1) qui porte les électrodes (2, 2'), la couche de matériau M1 et la couche de matériau M2, est constitué par un matériau isolant intrinsèque ou par un matériau recouvert d'un matériau diélectrique.

4. Transducteur selon la revendication 3, **caractérisé en ce que** le matériau isolant est l'alumine ou le verre.

5. Transducteur selon la revendication 3, **caractérisé en ce que** le matériau recouvert d'un matériau diélectrique est constitué par du silicium recouvert par un film de silice ou de nitrure de silicium.

6. Transducteur selon la revendication 1, **caractérisé en ce que** les électrodes (2, 2') sont constituées par un film d'or ou par un film d'oxyde d'étain et d'indium (ITO).

7. Transducteur selon la revendication 6, **caractérisé en ce que** les électrodes (2, 2') sont sous forme d'électrodes interdigitées.

8. Capteur pour la détection d'espèces gazeuses acceptrices d'électrons ou donneuses d'électrons, **caractérisé en ce qu'**il comprend un transducteur selon l'une des revendications 1 à 7.

9. Capteur selon la revendication 8, **caractérisé en ce que** le matériau M1 est PcCu.

10. Capteur selon la revendication 8, **caractérisé en ce que** le matériau M1 est F₁₆PcCu.

11. Capteur selon la revendication 8, **caractérisé en ce que** le matériau M1 est F₈PcCu et les électrodes (2, 2') sont constituées par ITO ou Au.

## Patentansprüche

1. Messwandler, gebildet von einem isolierenden Substrat (1), auf dessen Oberfläche zwei Elektroden (2, 2') und ein sensibles halbleitendes Element aufgebracht sind, gebildet von einer Schicht eines halbleitenden molekularen Materials M1 im Kontakt mit den Elektroden (2, 2') und einer Schicht eines halbleitenden molekularen Materials M2, das auf der Materialschicht M1 aufgebracht ist und nicht im Kontakt mit den Elektroden (2, 2') ist, wobei der Messwandler **dadurch gekennzeichnet ist, dass**:
- das Material M1 ein Monophthalocyanin ist, ausgewählt aus PcCu, F₈PcCu und F₁₆PcPu; dem α,ω-Dihexylsexithiophen oder der N,N-Dipentylperylentetracarbondiimidsäure,
- das Material M2 Lu(Pc)₂ ist.

2. Messwandler nach Anspruch 1, **dadurch gekennzeichnet, dass** die Stärke jeder der Schichten M1, M2 und der Elektroden (2, 2') zwischen 2 und 1000 nm inklusive beträgt.

3. Messwandler nach Anspruch 1, **dadurch gekennzeichnet, dass** das isolierende Substrat (1), welches die Elektroden (2, 2'), die Materialschicht M1 und die Materialschicht M2 trägt, von einem intrinsischen isolierenden Material oder von einem Material gebildet ist, das von einer dielektrischen Schicht bedeckt ist.

4. Messwandler nach Anspruch 3, **dadurch gekennzeichnet, dass** das isolierende Material das Aluminiumoxid oder das Glas ist.

5. Messwandler nach Anspruch 3, **dadurch gekennzeichnet, dass** das mit einem dielektrischen Material bedeckte Material von Silizium, bedeckt mit einer Folie aus Siliziumdioxid oder Siliziumnitrid, gebildet ist.

6. Messwandler nach Anspruch 1, **dadurch gekennzeichnet, dass** die Elektroden (2, 2') von einer Goldfolie oder von einer Folie aus Zinn- und Indiumoxid (ITO) gebildet sind.

7. Messwandler nach Anspruch 6, **dadurch gekennzeichnet, dass** die Elektroden (2, 2') in Form ineinandergreifender Elektroden vorliegen.

8. Sensor für die Detektion von reduzierenden oder oxidierenden Gaskomponenten, **dadurch gekennzeichnet, dass** er einen Messwandler nach einem der Ansprüche 1 bis7 umfasst.

9. Sensor nach Anspruch 8, **dadurch gekennzeichnet, dass** das Material M1 PcCu ist.

10. Sensor nach Anspruch 8, **dadurch gekennzeichnet, dass** das Material M1 F₁₆PcCu ist.

11. Sensor nach Anspruch 8, **dadurch gekennzeichnet, dass** das Material M1 F₈PcCu ist und die Elektroden (2, 2') von ITO oder Au gebildet sind.

## Claims

1. A transducer made up of an insulating substrate (1) on the surface of which are deposited two electrodes (2, 2') and a sensitive semi-conductor element made up of a layer of a molecular semi-conductor material M1 in contact with the electrodes (2, 2') and a layer of a molecular semi-conductor material M2 that is deposited on the layer of material M1 and is not in contact with the electrodes (2, 2'), said transducer being **characterized in that**:
- the material M1 is a monophthalocyanine chosen from among PcCu, F₈PcCu and F₁₆PcCu; α,ω-dihexyl-sexithiophene or N,N-dipentyl-perylene tetracarboxylic diimide acid;
- the material M2 is Lu(Pc)₂.

2. The transducer according to claim 1, **characterized in that** the thickness of each of the layers M1, M2 and the electrodes (2, 2') is comprised between 2 and 1000 nm.

3. The transducer according to claim 1, **characterized in that** the insulating substrate (1) that bears the electrodes (2, 2'), the layer of material (M1) and the layer of material M2 is made up of an intrinsic insulating material or a material covered with a dielectric material.

4. The transducer according to claim 3, **characterized in that** the insulating material is alumina or glass.

5. The transducer according to claim 3, **characterized in that** the material covered with a dielectric material is made up of silicon covered with a silica or silicon nitride film.

6. The transducer according to claim 1, **characterized in that** the electrodes (2, 2') are made up or a gold film or an indium tin oxide (ITO) film.

7. The transducer according to claim 6, **characterized in that** the electrodes (2, 2') are in the form of interdigitated electrodes.

8. A sensor for detecting electron acceptor- or electron donor-type gaseous species, **characterized in that** it includes a transducer according to one of claims 1 to 7.

9. The sensor according to claim 8, **characterized in that** the material M1 is PcCu.

10. The sensor according to claim 8, **characterized in that** the material M1 is F₁₆PcCu.

11. The sensor according to claim 8, **characterized in that** the material M1 is F₈PcCu, and the electrodes (2, 2') are made up of ITO or Au.
